# EUROPEAN PATENT SPECIFICATION

(11) **EP 2 012 836 B1**
(45) Date of publication and mention of the grant of the patent: **07.03.2012**
(21) Application number: 07708595.9
(22) Date of filing: 25.01.2007
(51) Int. Cl.: A61L 2/06, A61L 2/07, A47J 27/04

(54) **HEATING, STERILIZING AND DRYING APPLIANCE USING SUPERHEATED STEAM GENERATOR**
ERWÄRMUNGS-, STERILISATIONS- UND TROCKNUNGSGERÄT MIT EINEM GENERATOR ZUR ERZEUGUNG ÜBERHITZTEN DAMPFES
APPAREIL DE CHAUFFAGE, DE STÉRILISATION ET DE SÉCHAGE UTILISANT UN GÉNÉRATEUR DE VAPEUR SURCHAUFFÉE

(30) Priority: 28.04.2006 KR 20060038612; 13.10.2006 KR 20060099833
(43) Date of publication of application: 14.01.2009
(73) Proprietor: Jaeyoung Solutec Co., Ltd, Namdong-gu, Incheon 405-300 (KR)
(72) Inventor: KIM, Hak Kwon, Seoul 137-857 (KR)
(74) Representative: Goddar, Heinz J.
(86) International application number: PCT/KR2007/000433
(87) International publication number: WO 2007/126197

(56) References cited:
- WO-A1-2004/005798
- WO-A1-2007/069855
- JP-A- 2003 014 203
- JP-A- 2006 105 523
- KR-B1- 100 644 867
- US-A- 620 994
- US-A- 756 840
- US-A- 2 616 023
- US-A- 2 779 315

## Description

### Technical Field

The present invention relates to a heating, sterilizing and drying appliance using a superheated steam generator, which uses, as a heat source, superheated steam produced from the superheated steam generator, and more particularly, to such a heating, sterilizing and drying appliance which reduces a manufacturing cost by simplifying the structure of the superheated steam generator, minimizes the size of steam molecules to produce high-temperature steam which is easy to permeate into a to-be heated material to maximize a temperature-raising effect.

### Background Art

In general, a superheated steam generator refers to a device which heats saturated steam supplied from a boiler and so forth so as to produce a large quantity of superheated steam during a short time period. The superheated steam produced by the superheated steam generator is used as a heat source of food cooking equipment, sterilizer equipment or drier equipment, since it is made of relatively high-temperature, fine particles as compared to the saturated steam.

For instance, the cooking equipment using the superheated steam has a merit that it is characterized by uniformly heating food so that the food is not burnt locally and the temperature and the supply amount of the superheated steam are easily adjusted to readily control the cooking characteristic, as well as oxidation due to the steam is prevented to allow the original flavor to be maintained.

However, the conventional superheated steam generator entails demerits in that since it has a plurality of complicated constituent parts including a screw-type heat transfer tubes embedded therein so as to secure a fluid-passing cross-section required for generating and treating the superheated steam, a manufacturing cost increases, which leads to an increase in maintenance and repair costs of products.

Furthermore, the conventional superheated steam generator encounters a problem in that it is difficult to obtain superheated steam having a uniform temperature since a deviation of steam flow rate is large due to a complexity of built-in components. Moreover, if local overheat occurs in the heat transfer tube itself due to the deviation of fluid flow rate, there is caused another problem in that the built-in components are deteriorated to thereby degrade their durability.

US 756,840 discloses an apparatus for superheating steam comprising a combination of a furnace, a main steam generator therein, a consuming device provided with a superheating chamber and an auxiliary steam generator provided with circulating conductors extending into the path of the furnace gases in their course to the main steam generator.

US 2,779,315 describes a steam generator for producing high pressure superheated steam comprising a cylindrical casing, a burner in the casing, a plurality of superposed pairs of plates mounted in the casing and a plurality of pipes providing communication between adjacent plates. Conical diffusers provide a circuitous pathway for hot gases from the burner. A high pressure pump and a nozzle are provided for injecting water vapor into the casing.

### Disclosure of Invention

### Technical Problem

Accordingly, the present invention has been made to address and solve the above-mentioned problems occurring in the prior art, it is an object of the present invention to provide a heating, sterilizing and drying appliance using a superheated steam generator, which reduces a manufacturing cost by simplifying the structure of the superheated steam generator, minimizes the size of steam molecules to produce high-temperature steam which is easy to permeate into a to-be heated material to maximize a temperature-raising effect.

### Technical Solution

To accomplish the above object, according to one aspect of the present invention, there is provided a heating appliance according to claim 1.

### Advantageous Effects

According to the heating appliance using a superheated steam of the present invention, the saturated steams supplied to the superheated steam generator is heated while generating vortices by the unique construction of the superheated steam generator so that they are decomposed to small-sized molecules (clusters) to thereby improve the temperature efficiency of the superheated steam.

Further, the time and cost spent for generating the superheated steam is minimized, and the inner construction of the superheated steam generator is simplified to thereby reduce various expenses according to manufacture, maintenance and repair.

### Brief Description of the Drawings

FIG. 1 is a conceptual view illustrating a heating, sterilizing and drying appliance using a superheated steam generator according to the present invention;

FIG. 2 is a top exploded perspective view illustrating the superheated steam generator according to the present invention;

FIG. 3 is a cross-sectional view illustrating the superheated steam generator according to the present invention in an assembled state;

FIGs. 4(a) and 4(b) are cross-sectional views illustrating a heating element of the superheated steam generator according to the present invention; and

FIG. 5 is a cross-sectional view illustrating a superheated steam injection line of a superheated steam supply line according to the present invention.

### Best Mode for Carrying Out the Invention

Reference will now be made in detail to the preferred embodiment of the present invention with reference to the attached drawings.

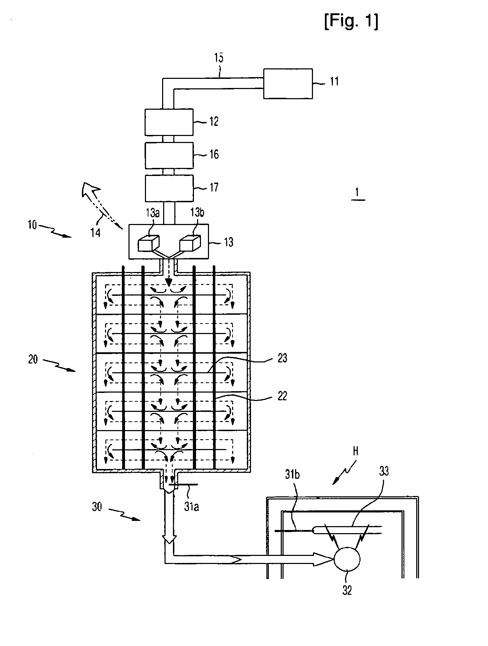

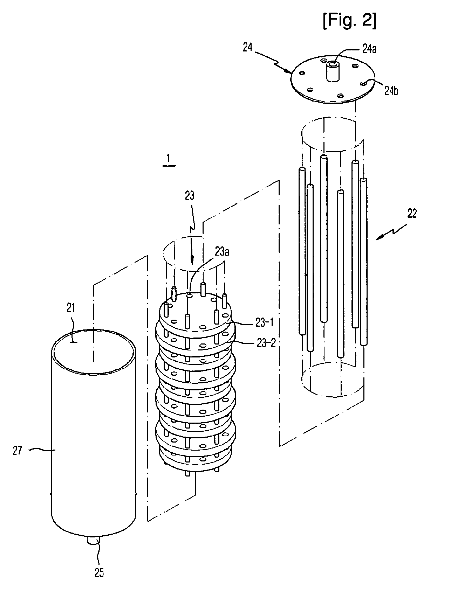

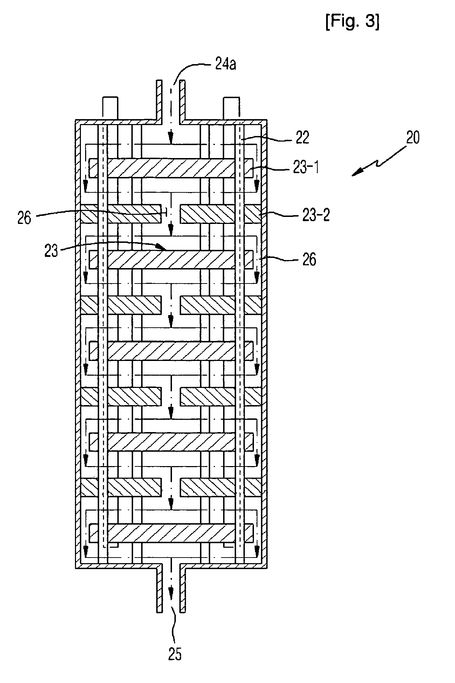

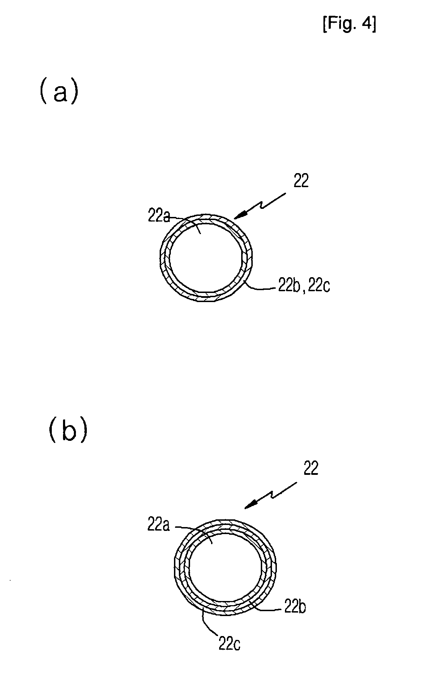

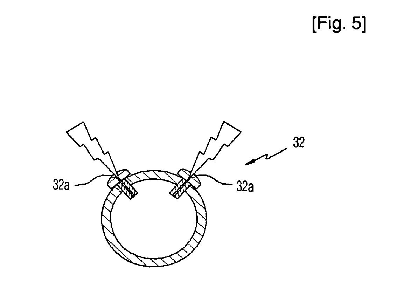

FIG. 1 is a conceptual view illustrating a heating, sterilizing and drying appliance using a superheated steam generator according to the present invention in a disassembled state, FIGs. 2 and 3 are top exploded perspective and cross-sectional views illustrating the superheated steam generator according to the present invention, FIGs. 4(a) and 4(b) are cross-sectional views illustrating a heating element of the superheated steam generator according to the present invention, and FIG. 5 is a cross-sectional view illustrating a superheated steam injection line of a superheated steam supply line according to the present invention.

The heating, sterilizing and drying appliance using a superheated steam generator according to the present invention includes: a saturated steam distributor 10 adapted to selectively distribute saturated steam which is produced by a boiler 11 and then is separated from condensed water while passing through a separator 12 in the process of opening a valve assembly 13; a superheated steam generator 20 adapted to allow streams of the steam supplied thereto from the saturated steam distributor 10 to generate vortices while passing through a plurality of disc-like interval-maintaining member 23 stackingly arranged on top of one another longitudinally in a combustion chamber 21 thereof and adapted to heat the vortex-generating steam through a plurality of superheated steam heating elements 22penetratingly mounted vertically in the plurality of interval-maintaining members to thereby produce superheated steam; and a superheated steam supply line 30 adapted to inject the superheated steam which is produced by the superheated steam generator 20 and is discharged after passing through the combustion chamber 21 toward a heating place H through a superheated steam injection line 32.

First, the appliance using a superheated steam generator according to the present invention is constructed of a structure such as a heating appliance, a sterilizing appliance and a drying appliance. Since the constituent elements such as the heating appliance, the sterilizing appliance and the drying appliance are identical to one another, they are denoted and described by using the same reference numerals. Specifically, the present invention will be described hereinafter while being limited to the heating appliance among all the appliances.

Here, the heating appliance 1 is constructed of a saturated steam distributor 10 for distributing saturated steam which is produced by a boiler 11, a superheated steam generator 20 for heating the steam supplied thereto from the saturated steam distributor 10 to thereby produce superheated steam, a superheated steam supply line 30 for supplying the superheated steam which is produced by the superheated steam generator 20 to a use place (heating place), and a controller (not shown) for controlling the heating of the constituent elements.

The saturated steam distributor 10 includes a saturated steam supply line 15 connected to a boiler 11 so as to allow saturated steam produced from the boiler 11 to be supplied therethrough, a separator 12 mounted on a pipe path of the saturated steam supply line 15 so as to separate water and the saturated steam from each other, and a valve assembly 13 for adjusting the supply amount of the saturated steam separated by the separator 12.

Also, on the saturated steam supply line 15 is mounted a drain tube 14 for discharging the condensed water and the residual saturated steam remained after being distributed by the valve assembly 13 and supplied to the superheated steam generator 20.

In addition, on the saturated steam supply line 15 are mounted a pressure-reducing valve 16 for reducing a pressure of the saturated steam supplied from the boiler 11 to the pressure level needed by the superheated steam generator 20, and a safety valve 17 for controlling the vapor pressure of the saturated steam to be introduced into the superheated steam generator 20 to be a prescribed pressure.

Further, the valve assembly 13 serves to adjust the amount of the saturated steam supplied from the boiler 11 for supply to the superheated steam generator 20, and consists of first and second electronic valves 13a and 13b whose capacities are different from each other. The valve assembly 13 is configured such that the first electronic valve 13a and the second electronic valve 13b are opened/closed concurrently or separately in response to a control signal generated from a controller (not shown) so as to adjust the amount of the saturated steam to be introduced into the superheated steam generator 20.

In addition, the superheated steam generator 20 has been invented by applying " a superheated steam generating device (Korean Application No. 10-2005-123156) which was filed prior to the filing of the present application by the applicant of the present invention. The superheated steam generator 20 includes a housing 27 formed with a combustion chamber 21 for therein accommodating the saturated steam selectively supplied by the valve assembly 13, a plurality of disc-like interval-maintaining members 23 stackingly arranged on top of one another longitudinally in the combustion chamber 21 of the housing 27, and a plurality of superheated steam heating elements 22 penetratingly mounted vertically in the plurality of disc-like interval-maintaining members 23so as to produce superheated steam.

Also, the housing 27 has a discharge outlet 25 formed at a bottom end thereof so as to allow superheated steam to be discharged to the superheated steam supply line 30 therethrough, and a cover 24 is mounted on a top end of the combustion chamber 21 of the housing 27 and has an injection inlet 24a formed at a top end thereof to be connected to the saturated steam supply line 15 so as to allow the saturated steam to be injected into the combustion chamber 21 therethrough.

At this time, the housing 27 is preferably formed in a cylindrical or polygonal shape, and particularly is preferably made of a material excellent in corrosion resistance and heat resistance such as stainless steel, etc.

In addition, the cover 24 is preferably securely fixed to the top end portion of the combustion chamber 21 of the housing 27 by means of a fastening means such as a bolt or in a press-fitting manner. Specifically, the cover 24 has a plurality of through-holes 24b formed thereon for allowing the plurality of superheated steam heating elements 22 to be protruded upwardly therethrough, which will be described later.

The plural disc-like interval-maintaining members 23 are horizontally stackingly arranged on top of one another in the combustion chamber 21 of the housing 27 in such a fashion as to be spaced apart from one another by equal distances, so that the disc-like interval-maintaining members 23 allows the stream of the superheated steam to generate vortices and serves to support the superheated steam heating elements 22. The disc-like interval-maintaining members 23-1 and 23-2 positioned in odd and even number rows are formed to be different from each other in outer diameters.

Also, the disc-like interval-maintaining members 23 allows a fluid-communicating passageway 26 to be defined in a gap between the inner wall of the combustion chamber 21 and the interval-maintaining members 23-1 of odd number rows, and at the central portion of the interval-maintaining members 23-2 of even number rows, respectively, so that the steam can passes therethrough while generating vortices. Of course, the fluid-communicating passageways 26 respectively formed in the disc-like interval-maintaining members 23-1 and 23-2 of even and odd number rows may be altered in position.

Also, the disc-like interval-maintaining members 23 can preferably be made of a material excellent in corrosion resistance and heat resistance such as stainless steel, etc., but it is natural that the interval-maintaining member 23 may be made of a ceramic material. Moreover, each of the disc-like interval-maintaining members 23 has a plurality of fitting holes 23a formed therein in such a fashion as to be arranged spaced apart from one another equidistantly at a peripheral portion of the disc-like interval-maintaining members so as to fixedly fit the superheated steam heating elements 22 thereto.

The superheated steam heating elements 22 are securely fit into the fitting holes 23a of the disc-like interval-maintaining members 23 by means of welding or press-fitting so as to heat steam which is supplied to the combustion chamber 21 and generates vortices while passing through the fluid-communicating passageways 26

Also, the superheated steam heating elements 22 includes stainless steel 22b or ceramic 22c coated on a hot wire 22a disposed at the central portion thereof, or the stainless steel 22b and ceramic 22c are sequentially coated on the hot wire 22a so as to protect the hot wire or emit a far-infrared ray.

At the heating place H is mounted a ceramic heater 33 made of a ceramic material for emitting a far-infrared ray with a high permeability so as to improve firing, a heating and drying speed in cooperation with the atomized superheated steam flow.

At this time, the ceramic heater 33 is of course used to prevent the condensation of the superheated steam injected through the superheated steam injection line 32 as well as adjust a heating temperature in the heating place H. Also, it is preferably mount an automatic temperature gauge 31b for measuring and adjusting the temperature of the ceramic heater 33 in the heating place H.

The superheated steam supply line 30 is configured such that the superheated steam produced by heating while vortices being generated by means of the interaction between the disc-like interval-maintaining members 23 and the superheated steam heating elements 22 is injected toward the heating place H using the superheated steam injection line 32. It is preferable to mount an automatic temperature gauge 31a in the discharge outlet line of the superheated steam generator 20 so as to measure and adjust the temperature of the superheated steam.

In addition, the superheated steam injection line 32 is configured such that a plurality of conical holes are directly drilled in a row on the outer surface of a body of the superheated steam injection line or round screws each having an injection hole 32a formed centrally therein are uniformly arranged on the outer surface of the body of the superheated steam injection line in such a fashion as to be juxtaposedly disposed at both sides of the outer surface of the body of the superheated steam injection line at an injection angle of 30 degrees.

That is, the superheated steam injection line 32, as shown in FIG. 5, has round screws made of stainless steel disposed at both sides of the outer surface of the body thereof. In this case, it is preferable to form conical injection holes 32a having an angle and length conforming to the round screws.

As a result, if the hole of the superheated steam injection line 32 is blocked off due to foreign substances, only the round screws used are removed to thereby facilitate their replacement with new ones.

At this time, examples of the heating place H employing the superheated steam produced by the superheated steam supply line 30 include a cooking appliance used for cooking foods such as, for example, pizza, etc., various heating appliances, a sterilizing and drying appliance, a carbonizing heat-treatment apparatus for drying general wastes, paints, wood materials and the like, etc.

The controller (not shown) is electrically connected to the saturated steam distributor 10, the superheated steam generator 20 and the superheated steam supply line 30 so as to receive the pressure, the amount of the steam, the heating time and the temperature signal of the constituent parts to generate an operating signal therefrom.

Now, the operation of the heating, sterilizing and drying appliance according to the present invention will be described hereinafter.

First, in case of the heating appliance, the saturated steam produced by the boiler 11 of the saturated steam distributor 10 is set to a prescribed pressure by the pressure-reducing valve 16 and the safety valve 17, and then passes through the valve assembly 13 so as to be supplied to the superheated steam generator 20.

Then, pure saturated steam which has passed through the separator 12 is injected into the superheated steam generator 20 through the valve assembly 13. In the meantime, the pure saturated steam is separated into water and vapor by the separator 12, and the condensed water and the residual saturated steam which has not passed through the valve assembly 13 are discharged through the drain tube 14 for removal.

At this time, since the supply amount of the saturated steam which has passed through the valve assembly 13 is controlled by the concurrent or separate operation of the first and second electronic valves 13a and 13b, it is adjusted depending on the state of the heating appliance.

In the meantime, the saturated steam supplied to the superheated steam generator 20 collides against the disc-like interval-maintaining members 23 or its molecules collide with one another to generate vortices while instantaneously passing through the fluid-communicating passageways 26 formed in the disc-like interval-maintaining members 23-1 and 23-2 of odd and even number rows.

At this time, the superheated steams passing through the fluid-communicating passageways 26 is heated by the superheated steam heating elements 22 to generate vortices, so that they are decomposed to small-sized molecules (clusters) while colliding with one another or the disc-like interval-maintaining members.

For this reason, the saturated steam becomes a superheated steam with permeability to thereby efficiently raise the temperature of the superheated steam.

That is, when the steam heated by the superheated steam heating elements 22 is vaporized, it needs an energy of 539 cal/g to thereby hold latent heat of vaporation. Thus, as the particles of the steam are smaller, they are more easily permeated to a to-be-heated object.

At this time, in case where a far-infrared ray ceramic heater 33 is further mounted at the heating place H, a typical shortcoming of the superheated steam, i.e., a phenomenon can be prevented where the superheated steam is condensed due to a cooling effect occurring immediately after being injected toward the heating place H.

Furthermore, since a far-infrared ray emitted from the ceramic heater 33 on which ceramic material is coated and latent heat which the atomized superheated steam has are utilized together, firing, heating or drying of a to-be-heated object is performed more rapidly to thereby improve a heating efficiency.

In addition, the superheated steam heating elements 22 of the superheated steam generator 20 is turned off so as to allow the saturated steam to pass through the first and second electronic valves 13a and 13b so that the saturated steam can be utilized as steam for cleaning purpose.

### Industrial Applicability

As described above, according to the heating, sterilizing and drying appliance using a superheated steam generator of the present invention, the saturated steams supplied to the superheated steam generator is heated while generating vortices by the unique construction of the superheated steam generator so that they are decomposed to small-sized molecules (clusters) to thereby improve the temperature efficiency of the superheated steam.

Further, the time and cost spent for generating the superheated steam is minimized, and the inner construction of the superheated steam generator is simplified to thereby reduce various expenses according to manufacture, maintenance and repair.

Moreover, with the combined use of a far-infrared ray ceramic heater, a typical shortcoming of the superheated steam, i.e., a rapid drop in temperature or condensation is prevented. Utilization of a temperature-increasing effect of the far-infrared ray and the latent heat of vaporation which the superheated steam holds can remarkably reduce the time required for heating for cooking, drying and sterilizing.

In addition, in case where the injection holes formed at the superheated steam injection line is clogged during the operation of the superheated steam generator, only the round screws used can be replaced with new ones without a need of exchanging the whole superheated steam injection line, thereby reducing various expenses according to the maintenance and repair of the system.

While the invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.

## Claims

1. A heating appliance using a superheated steam generator, comprising:
a saturated steam distributor (10) comprising a boiler (11), a separator (12) and a valve assembly 13;
a superheated steam generator (20) adapted to allow streams of the steam supplied thereto from the saturated steam distributor to generate vortices while passing through a plurality of disc-like interval-maintaining means (23) stackingly arranged on top of one another longitudinally in a combustion chamber (21) thereof and adapted to heat the vortex-generating steam through a plurality of superheated steam heating means (22) penetratingly mounted vertically in the plurality of interval-maintaining means to thereby produce superheated steam; and
a superheated steam supply line (30) adapted to inject the superheated steam which is produced by the superheated steam generator (20) and is discharged after passing through the combustion chamber (21) toward a heating place (H) through a superheated steam injection line (32).

2. The heating appliance according to claim 1, wherein the valve assembly (13) includes at least one pair of electronic valves whose capacities for allowing the saturated steam to pass therethrough are different from each other so as to be opened/closed concurrently or separately.

3. The heating appliance according to claim 1, wherein the plural disc-like interval-maintaining means (23) is configured such that disc-like interval-maintaining means (23-1 and 23-2) positioned in odd and even number rows are formed to be different from each other in outer diameters, and a fluid-communicating passageway (26) is defined in a gap between the inner wall of the combustion chamber (21) and the interval-maintaining means (23-1) of odd number rows, and at the central portion of the interval-maintaining means (23-2) of even number rows, respectively, so that the steam can passes therethrough while generating vortices.

4. The heating appliance according to claim 1, wherein the superheated steam heating means (22) includes ceramic (22b) or stainless steel (22c) coated on a hot wire (22a) disposed at the central portion thereof.

5. The heating appliance according to claim 1, wherein the superheated steam heating means (22) includes ceramic(22b)and stainless steel (22c) sequentially coated on a hot wire (22a) disposed at the central portion thereof.

6. The heating appliance according to claim 1, wherein the superheated steam injection line (32) is configured such that a plurality of conical holes are directly drilled in a row on the outer surface of a body of the superheated steam injection line or round screws each having an injection hole (32a) formed centrally therein are uniformly arranged on the outer surface of the body of the superheated steam injection line in such a fashion as to be juxtaposedly disposed at both sides of the outer surface of the body of the superheated steam injection line.

7. the heating appliance according to claim 1, wherein an automatic temperature gauge (31a) is mounted in the discharge outlet line of the superheated steam generator (20) so as to measure and adjust the temperature of the superheated steam.

8. The heating appliance according to claim 1, wherein a ceramic heater (33) is mounted at the heating place (H) so as to prevent the condensation of the superheated steam injected through the superheated steam injection line (32) as well as adjust a heating temperature in the heating place (H).

9. The heating appliance according to claim 8, wherein an automatic temperature gauge (31b) is mounted at the heating place (H) so as to measure and adjust the temperature of the ceramic heater (33).

## Patentansprüche

1. Beheizungsvorrichtung, bei der ein Dampferzeuger für überhitzten Dampf verwendet wird, umfassend
einen Verteiler (10) für überhitzten Dampf, umfassend einen Heizkessel (11), einen Separator (12) und eine Ventilanordnung (13);
einen Dampferzeuger (20) für überhitzten Dampf, der eingerichtet ist, um zu ermöglichen, dass Ströme des Dampfs, die diesem von dem Verteiler für überhitzten Dampf zugeführt werden, Wirbel erzeugen, während sie durch eine Vielzahl von scheibenartigen abstandhaltenden Mitteln (23) hindurchgehen, die stapelartig übereinander in Längsrichtung in einer Brennkammer (21) davon angeordnet sind und eingerichtet sind, den Wirbel erzeugenden Dampf durch eine Vielzahl von Beheizungsmitteln (22) für überhitzten Dampf zu erhitzen, die durchdringend vertikal in der Vielzahl von abstandhaltenden Mittel angebracht sind, um dadurch überhitzten Dampf zu erzeugen; und
eine Zufuhrleitung (30) für überhitzen Dampf, die eingerichtet ist, um den überhitzten Dampf, der von dem Dampferzeuger (20) für überhitzten Dampf erzeugt worden ist und nach Durchgang durch die Brennkammer (21) abgegeben wird, zu einer Beheizungsstelle (H) über eine Einspritzleitung (32) für überhitzten Dampf einzuspritzen.

2. Beheizungsvornchtung nach Anspruch 1, **dadurch gekennzeichnet, dass** die Ventilanordnung (13) zumindest ein Paar von elektronischen Ventilen umfasst, deren Kapazitäten, um überhitzten Dampf durch diese hindurchzuleiten, sich voneinander unterscheiden, so dass sie gleichzeitig oder getrennt geöffnet bzw. geschlossen werden.

3. Beheizungsvorrichtung nach Anspruch 1, **dadurch gekennzeichnet, dass** die mehreren scheibenartigen abstandhaltenden Mittel (23) so ausgebildet sind, dass scheibenartige abstandhaltende Mittel (23-1 und 23-2), die in geradzahligen und ungeradzahligen Reihen angeordnet sind, so ausgebildet sind, dass sie sich in ihren äußeren Durchmessern voneinander unterscheiden, und wobei ein eine Fluidverbindung herstellender Durchgang (26) in einem Spalt zwischen der inneren Wand der Brennkammer (21) und den abstandhaltenden Mitteln (23-1) der ungeradzahligen Reihen festgelegt ist, und an dem mittleren Abschnitt der abstandhaltenden Mittel (23-2) der geradzahligen Reihen, so dass der Dampf dazwischen durchgehen kann, während Wirbel erzeugt werden.

4. Beheizungsvorrichtung nach Anspruch 1, **dadurch gekennzeichnet, dass** die Beheizungsmittel (22) für den überhitzten Dampf Keramik (22b) oder rostfreien Stahl (22c) umfassen, die bzw. der auf einem heißen Draht (22a) beschichtet ist, der in dem mittleren Bereich davon angeordnet ist.

5. Beheizungsvorrichtung nach Anspruch 1, **dadurch gekennzeichnet, dass** die Beheizungsmittel (22) für den überhitzten Dampf Keramik (22b) und rostfreien Stahl (22c) umfassen, die der Reihe nach auf einen heißen Draht (22a) beschichtet sind, der an dem mittleren Bereich davon angeordnet ist.

6. Beheizungsvornchtung nach Anspruch 1, **dadurch gekennzeichnet, dass** die Einspritzleitung (32) für den überhitzten Dampf so konfiguriert ist, dass eine Vielzahl von konischen Öffnungen unmittelbar in einer Reihe auf der äußeren Oberfläche eines Körpers der Einspritzleitung für den überhitzten Dampf gebohrt sind, oder runde Schrauben, die jeweils mit einer Einspritzöffnung (32a) versehen sind, die zentral darin ausgebildet ist, gleichmäßig auf der äußeren Oberfläche des Körpers der Einspritzleitung für den überhitzten Dampf angeordnet sind, auf eine solche Weise, dass sie an beiden Seiten der äußeren Oberfläche des Körpers der Einspritzleitung für den überhitzten Dampf nebeneinander angeordnet sind.

7. Beheizungsvornchtung nach Anspruch 1, **dadurch gekennzeichnet, dass** eine automatische Temperaturmesseinrichtung (31 a) in der Abgabe-Auslassleitung des Dampferzeugers (20) für überhitzten Dampf angebracht ist, um die Temperatur des überhitzten Dampfs zu messen und einzustellen.

8. Beheizungsvorrichtung nach Anspruch 1, **dadurch gekennzeichnet, dass** eine keramische Heizung (33) an der Beheizungsstelle (H) angebracht ist, um die Kondensation des überhitzten Dampfs zu verhindern, der durch die Einspritzleitung (32) für den überhitzten Dampf eingespritzt wird, und auch, um eine Beheizungstemperatur an der Beheizungsstelle (H) einzustellen.

9. Beheizungsvornchtung nach Anspruch 8, **dadurch gekennzeichnet, dass** eine automatische Temperaturmesseinrichtung (31b) an der Beheizungsstelle (H) angebracht ist, um die Temperatur der keramischen Heizung (33) zu messen und einzustellen.

## Revendications

1. Appareil de chauffage employant un générateur de vapeur surchauffée, et comprenant
un distributeur de vapeur saturée (10), qui comporte une chaudière (11), un séparateur (12) et un groupe de vannes (13);
un générateur de vapeur surchauffée (20) adapté de manière à ce que les flux de vapeur qui y sont alimentés et qui proviennent du distributeur de vapeur saturée produisent des tourbillons pendant leur passage à travers une pluralité de disques de maintien des intervalles (23) empilés les uns au-dessus des autres longitudinalement dans une chambre de combustion (21) du générateur, et adaptés de manière à chauffer la vapeur qui produit des tourbillons à travers une pluralité d'éléments de chauffage (22) à la vapeur surchauffée qui sont montés à la verticale à l'intérieur de la pluralité des disques de maintien des intervalles afin de produire de la vapeur surchauffée; et
une tuyauterie d'alimentation (30) de vapeur surchauffée adaptée de manière à injecter la vapeur surchauffée qui est produite par le générateur de vapeur surchauffée (20) et qui est déchargée après son passage à travers la chambre de combustion (21) pour se rendre à une unité de chauffage (H) en passant par une tuyauterie d'injection (32) de vapeur surchauffée.

2. Appareil de chauffage selon la revendication 1, **caractérisé en ce que** le groupe de vannes (13) comprend au moins deux vannes électroniques qui font passer la vapeur saturée à des débits différents, ceci permettant de les ouvrir et de les fermer en même temps ou bien séparément.

3. Appareil de chauffage selon la revendication 1, **caractérisé en ce que** la pluralité des disques de maintien des intervalles (23) est configurée de manière à ce que les disques (23-1 et 23-2), qui se trouvent placés dans les rangées de nombre impair et les rangées de nombre pair, ont des diamètres extérieurs différents, de manière aussi à ce qu'un canal de transfert de fluide (26) soit défini dans un intervalle entre la paroi interne de la chambre de combustion (21) et les disques de maintien des intervalles (23-1) des rangées de nombre impair, et au niveau de la partie centrale des disques de maintien des intervalles (23-2) des rangées de nombre pair, respectivement, ceci permettant à la vapeur de les traverser tout en créant des tourbillons.

4. Appareil de chauffage selon la revendication 1, **caractérisé en ce que** les éléments de chauffage à la vapeur surchauffée (22) comprennent une couche de céramique (22b) ou une couche d'acier inoxydable (22c) revêtue sur un fil chaud (22a) disposé au centre de l'appareil de chauffage.

5. Appareil de chauffage selon la revendication 1, **caractérisé en ce que** les éléments de chauffage à la vapeur surchauffée (22) comprennent une couche de céramique (22b) et une couche d'acier inoxydable (22c) revêtues séquentiellement sur un fil chaud (22a) disposé au centre de l'appareil de chauffage.

6. Appareil de chauffage selon la revendication 1, **caractérisé en ce que** la tuyauterie d'injection de vapeur surchauffée (32) est configurée de telle sorte qu'une pluralité d'orifices coniques soient directement percés le long d'une rangée sur la surface extérieure de la tuyauterie d'injection de vapeur surchauffée, ou bien que des vis rondes comportant chacune un orifice d'injection (32a) percé au centre soient disposées de manière uniforme sur la surface extérieure de la tuyauterie d'injection de vapeur surchauffée afin de se trouver juxtaposées sur les deux côtés de la surface extérieure de la tuyauterie d'injection de vapeur surchauffée.

7. Appareil de chauffage selon la revendication 1, **caractérisé en ce qu'**un indicateur de température automatique (31 a) est monté dans la tuyauterie de décharge du générateur de vapeur surchauffée (20) afin de mesurer et ajuster la température de la vapeur surchauffée.

8. Appareil de chauffage selon la revendication 1, **caractérisé en ce que** qu'un élément chauffant en céramique (33) est monté au niveau de l'unité de chauffage (H) de manière à empêcher la condensation de la vapeur surchauffée injectée par la tuyauterie d'injection de vapeur surchauffée (32), et aussi à ajuster la température de chauffage de l'unité de chauffage (H).

9. Appareil de chauffage selon la revendication 8, **caractérisé en ce qu'**un indicateur de température automatique (31b) est monté dans l'unité de chauffage (H) afin de mesurer et ajuster la température de l'élément chauffant en céramique (33).
